(19) Europäisches Patentamt
European Patent Office
Office européen des brevets

(11) Publication number: 0 134 668
A1

(12) # EUROPEAN PATENT APPLICATION

(21) Application number: 84304796.0

(22) Date of filing: 13.07.84

(51) Int. Cl.[4]: C 07 C 69/96
C 07 C 68/00

(30) Priority: 05.08.83 GB 8321199

(43) Date of publication of application:
20.03.85 Bulletin 85/12

(84) Designated Contracting States:
DE FR GB IT

(71) Applicant: IMPERIAL CHEMICAL INDUSTRIES PLC
Imperial Chemical House Millbank
London SW1P 3JF(GB)

(72) Inventor: Short, Glyn David
12 Fir Tree Close
Hilton Yarm Cleveland(GB)

(72) Inventor: Spencer, Michael Staines
Brookdale 189 Oxbridge Lane
Stockton-on-Tees Cleveland(GB)

(74) Representative: Gratwick, Christopher et al,
Imperial Chemical Industries PLC Legal Department:
Patents PO Box 6 Bessemer Road
Welwyn Garden City Herts, AL7 1HD(GB)

(54) **Carbonate production.**

(57) Continuous production of di-alkyl carbonates by the catalytic reaction of carbon monoxide and oxygen with an alcohol wherein the alcohol is introduced into the vessel containing the di-alkyl carbonate at substantially the rate at which it is consumed and, to maintain a low water content in the reaction vessel, part of the vessel contents are continuously withdrawn, water and di-alkyl carbonate are separated, and some di-alkyl carbonate is recycled so that the net rates of removal of water and di-alkyl carbonate from the vessel are approximately equal to their rate of formation.

EP 0 134 668 A1

Carbonate production

This invention relates to carbonate production and in particular to the manufacture of di-alkyl carbonates such as dimethyl carbonate.

It has been proposed in US-A-3952045 and US-A-4218391 to produce di-alkyl carbonates by reacting an alcohol with carbon monoxide and oxygen in the presence of a suitable catalyst, for example a copper compound, particularly a salt such as a cuprous halide, alone or in admixture with an organic phosphorus compound. In those prior references, batch processes are described wherein an autoclave containing the alcohol and the catalyst is pressurised with a mixture of carbon monoxide and oxygen and the mixture heated to effect reaction.

During the reaction water is produced in accordance with the equation

$$2ROH + CO + \tfrac{1}{2}O_2 \longrightarrow (RO)_2CO + H_2O,$$

where R is an alkyl group

It has been found that as a result the conversion and yield that can be achieved are limited since, as the water content increases, the catalyst becomes de-activated and the reaction becomed less selective giving rise to undesired by-products such as carbon dioxide.

We have now devised a continuous process wherein these difficulties are overcome.

Accordingly the present invention provides a process for the production of a di-alkyl carbonate in which an alcohol

is reacted with carbon monoxide and oxygen in the presence of a catalyst, characterised in that the reaction is performed continuously by performing the following steps in continuous or intermittent fashion,

(i) introducing the alcohol into a reaction vessel containing a mixture of the corresponding di-alkyl carbonate and the catalyst and to which carbon monoxide and oxygen are supplied and

(ii) removing from the vessel a first composition comprising water and said di-alkyl carbonate and,

(iii) recycling to said vessel a second composition resulting from separation of at least part of the water and di-alkyl carbonate from said first composition,

the rate of removal of said first composition from said vessel and the rate of recycle of said second composition being such that the net rates of removal of water and di-alkyl carbonate from the vessel are approximately equal to their rate of formation whereby

(a) the water content of the vessel contents is maintained at not more than 5% by weight, and

(b) the volume of liquid in the vessel remains substantially constant,

said alcohol being introduced substantially at the rate at which it is consumed so that the average alcohol content of the vessel contents is maintained at not more than 25% by weight.

To avoid deactivation of the catalyst, and to reduce side reactions, it is necessary that the net rate of removal of water from the vessel is such that the water content of the vessel contents is kept low, below 5, and preferably below 1%, by weight.

It is of course not essential that the rate of removal of the first composition and the rate of recycle of the second composition is such that the net rate of removal of di-alkyl carbonate and water exactly equal their rates of production in the short term. However in the long term operation, e.g. over a

time interval of say 6 hours, there should be such a balance.

The first composition is preferably removed from the vessel by distillation. For example where the reaction is effected at an elevated temperature, part of the gas phase may be bled from the vessel and the first composition condensed therefrom. Alternatively part of the liquid phase may be withdrawn from the vessel as the first composition.

The first composition comprises the desired di-alkyl carbonate product, in admixture with water. In the di-alkyl carbonate forming reaction, in the absence of side reactions, the di-alkyl carbonate and water are formed in equimolar amounts. However generally the first composition will not contain the di-alkyl carbonate and water in equimolar amounts but rather will contain an excess of di-alkyl carbonate. Consequently to ensure removal of the water produced in the reaction it is generally necessary to remove the first composition at such a rate that the di-alkyl carbonate is removed from the vessel at a rate greater than that at which it is produced. It is therefore necessary to return some di-alkyl carbonate to the reaction vessel so that the net rates of removal of water and di-alkyl carbonate are approximately equal to their rates of formation.

It is therefore necessary to separate from the first composition an amount of water and di-alkyl carbonate corresponding to the amounts thereof formed and to recycle the surplus di-alkyl carbonate.

The possibility of side reactions is also reduced by the use of low alcohol concentrations. The alcohol is introduced to the vessel substantially at the rate at which it is consumed so that the average alcohol concentration of the vessel contents is maintained at below 25, preferably less than 15, and in particular less than 5, % by weight. The optimum alcohol concentration will depend on the variety of factors, e.g. reaction temperature, pressure, and the nature of the catalyst: in general the requisite alcohol concentration bears an inverse relation to the catalyst activity; i.e. higher alcohol

concentrations are required when using less active catalysts than when using more active catalysts under otherwise identical conditions.

The use of low alcohol concentrations also has the advantage, particularly with low boiling alcohols such as methanol or ethanol, that the first composition will contain a relatively small proportion of the alcohol. This facilitates removal of the water from the vessel, especially where the first composition is removed by distillation. For example, considering the case where the alcohol is methanol, producing di-methyl carbonate, the lowest boiling component of the first composition is the methanol/di-methyl carbonate azeotrope which, at a pressure of 10 bar absolute contains approximately 5% by weight of di-methyl carbonate. If a large proportion of methanol was present in the vessel, in order to remove the water from the vessel, the first composition, which would comprise a relatively large proportion of the methanol/di-methyl carbonate azeotrope, would have to be removed at a high rate and a great deal thereof would need to be recycled. If however the proportion of methanol in the vessel is low, the first composition will consist of the methanol/di-methyl carbonate azeotrope with a much larger proportion of the di-methyl carbonate/water azeotrope, and so the rate at which the first composition is removed from the vessel can be reduced.

Where the first composition contains a significant proportion of the alcohol, it may be desirable, in the interests of feedstock economy, to recycle at least part of an alcohol containing fraction, separated from the first composition e.g. by distillation, to the reaction vessel as part of the alcohol feed thereto. The first composition will thus generally contain a mixture of the di-alkyl carbonate, water, and the alcohol. As mentioned above the alcohol can often be separated from the first composition, if desired, by distillation, usually as an azeotrope with the di-alkyl carbonate, and this can be recycled, e.g. as part or in some cases all, of the second composition.

Generally however the second composition, i.e. that which is to be recycled, will need to contain a larger amount of di-alkyl carbonate than is obtained by separation of the alcohol/di-alkyl carbonate azeotrope from the first composition.

It is not necessary however that the second composition is completely free of water: thus, for economic reasons, it may be desirable to recycle as the second composition a mixture of di-alkyl carbonate and some water. In this case of course it is necessary that the rate of removal of the first composition is adequate to extract from the vessel water at a rate approximately equal to the rate at which it is formed plus the rate at which it is recycled, so that the water content of the vessel contents is sufficiently low to avoid de-activation of the catalyst.

The second composition can be obtained from the first composition by distillation of the first composition to obtain a fraction that is richer in di-alkyl carbonate than the first composition: this fraction can be used as the second composition. Some or all of the heat required for this distillation may be supplied by the heat evolved during the exothermic di-alkyl carbonate forming reaction. Alternatively, or additionally, other methods may be employed to obtain a di-alkyl carbonate rich fraction for recycle.

In some cases, particularly where the alcohol content is low, the first composition may itself separate into water rich and di-alkyl carbonate rich phases: in other cases phase separation, giving a water rich phase and a di-alkyl carbonate rich phase, may only occur after removal of an alcohol containing fraction from the first composition. For example a di-methyl carbonate/water mixture can separate, at room temperature, into a water rich phase containing about 3% by weight of di-methyl carbonate and a more dense di-methyl carbonate rich phase containing about 89% by weight of di-methyl carbonate. As mentioned above, for economic reasons, it may be desirable to recycle some of the di-alkyl carbonate rich phase without separation of the water therefrom.

Where distillation is employed to effect separation of water from the first composition, the compositions of the azeotropes, e.g. between the di-alkyl carbonate and the alcohol and/or between the di-alkyl carbonate and water in the first composition will depend on the pressure. By selection of appropriate pressures the amount of di-alkyl carbonate that has to be recycled can be minimised. Likewise the compositions of liquid phases obtained by phase separation will depend on the temperature of separation.

The reaction is conveniently performed at temperatures within the range 100 to 250°C under at least sufficient pressure to maintain the presence of a liquid phase in the vessel. Preferably pressures in the range 10 to 120 bar absolute are employed. In some cases the reaction vessel may also contain an inert high boiling solvent. The use of such a high boiling solvent may enable higher reaction temperature to be employed without requiring unduly high pressures to maintain the presence of a liquid phase.

The catalyst employed is conveniently selected from those described in the art for example in aforesaid US-A-3952045 and US-A-4218391. Preferably the catalyst is a copper chloride system. The catalyst may be supported on a suitable support material: preferably the catalyst, or support bearing the catalyst, is employed in a sufficiently finely divided form that it is readily maintained in suspension in the liquid.

A portion of the catalyst may be removed from the vessel, e.g. in admixture with the liquid phase, continuously or intermittently and replaced by fresh catalyst or by catalyst that has undergone an appropriate regeneration procedure.

The alcohol employed is preferably a lower alcohol containing 1 to 5 carbon atoms and in particular is methanol or ethanol, giving rise to di-methyl and di-ethyl carbonates respectively. Methanol is the preferred alcohol.

The carbon monoxide and oxygen (which may, but less preferably, be in the form of air) are preferably introduced

into the liquid as fine bubbles. This provides agitation of the liquid not only to maintain the catalyst in suspension and to provide distribution of the carbon monoxide and oxygen throughout the liquid but also avoids localised high concentrations of methanol in the region, or regions, of methanol introduction.

The carbon monoxide and oxygen are preferably introduced to the reaction vessel separately to avoid handling explosive gas mixtures outside the vessel. Where the first composition is removed from the vessel by distillation, generally unreacted gas will also be removed with the di-alkyl carbonate, water, and alcohol. Some or all of the unreacted gas may be recycled to the reaction vessel.

As described in US-A-4318862 the carbon monoxide may be employed in the form of a mixture with hydrogen, e.g. a synthesis gas, so that a hydrogen enriched synthesis gas is produced as a byproduct. Similarly, if air is used to supply the oxygen, and synthesis gas to provide the carbon monoxide, the byproduct gas will contain a hydrogen/nitrogen mixture which may be used, for example, as a feedstock for ammonia production.

The invention is illustrated by the following example.

A gas mixture of 10% $^{v}/v$ carbon monoxide, 5% $^{v}/v$ oxygen, and 85% $^{v}/v$ nitrogen was introduced at a pressure of 30 bar absolute at a rate of 2 litres/hour into the liquid phase within a stirred 1 litre capacity Inconel lined reaction vessel containing 550 g of di-methyl carbonate and 100 g of a cupric (methoxy) chloride, ie $Cu^{II}$ (OMe)Cl catalyst. The vessel was maintained at 120°C and at a pressure of 30 bar absolute by means of a relief valve in the gas exit line. Methanol was pumped into the liquid phase in the vessel at a rate of 16 ml/hour. The rate of addition of carbon monoxide and oxygen was thus in a slight excess of that required to produce di-methyl carbonate from the methanol.

Before passing through the relief valve, the exit gases were passed through a water cooled condenser and the condensed liquid collected in a reservoir. The condensate could be

drawn off from the reservoir for refining.

After 3 hours continuous operation the amount of condensate obtained was about 77 g and contained about 84% $^{w}/w$ di-methyl carbonate, 12% $^{w}/w$ water and 4% $^{w}/w$ methanol. The liquid phase in the reaction vessel had a methanol content below 0.5% $^{w}/w$ and a water content below 1% $^{w}/w$. The amount of condensate collected corresponded to a rate of removal of "first composition", i.e. the condensate, from the vessel at a rate of about 24 ml/hr. Since the condensate contained about 84% $^{w}/w$ of di-methyl carbonate, di-methyl carbonate was being removed at a rate of about 22 g/hour. The rate of removal of condensate from the vessel is a small excess of that required to remove all the water produced in the reaction. Since the rate of production of di-methyl carbonate from the methanol was about 15 g/hour, it is seen that the rate of removal of removal of di-methyl carbonate exceeds its rate of formation by about 7 g/hour. Thus in order to maintain the amount of liquid in the reaction vessel approximately constant, it is thus seen that di-methyl carbonate has to be recycled at a rate corresponding to about 7 g/hour.

In this example, the excess rate of removal of di-methyl carbonate, viz approx. 7 g/hour, is relatively small so that the amount of di-methyl carbonate in the vessel over the 3 hour period has dropped by less than 5% $^{w}/w$. It is thus only necessary to replenish the di-methyl carbonate in the vessel only periodically. Desirably the replenishment of di-methyl carbonate in the vessel is performed at such a frequency that the amount of di-methyl carbonate therein does not vary more than about $\pm$ 10% $^{w}/w$ from a mean value.

The required amount of di-methyl carbonate to effect replenishment can be obtained from the condensate by a suitable distillation procedure to give a sufficient amount of di-methyl carbonate or of a di-methyl carbonate/water mixture containing only a small amount of water. In the latter case, where a di-methyl carbonate/water mixture is recycled, it will be

necessary to increase the rate of removal of the first composition, i.e. the condensate, from the vessel so that the net amount of water removed is approximately equal to the amount produced by the reaction.

In this mode of operation, the rate of removal of the first composition can be increased, so as to ensure net removal of the water produced, by increasing the gas flowrate: if the gas flow rate is increased however, to avoid too great an excess of the reactant gases, with consequent risk of the side reaction of oxidation of carbon monoxide to carbon dioxide, the proportion of inert diluent gas, e.g. nitrogen, in the gas mixture can be increased.

PA/CG/MP
6 July 1984

1. A process for the production of a di-alkyl carbonate in which an alcohol is reacted with carbon monoxide and oxygen in the presence of a catalyst, characterised in that the reaction is performed continuously by performing the following steps in continuous or intermittent fashion.

(i) introducing the alcohol into a reaction vessel containing a mixture of the corresponding di-alkyl carbonate and the catalyst and to which carbon monoxide and oxygen are supplied, and

(ii) removing from the vessel a first composition comprising water and said di-alkyl carbonate and

(iii) recycling to said vessel a second composition resulting from separation of at least part of the water and di-alkyl carbonate from said first composition,

the rate of removal of said first composition from said vessel and the rate of recycle of said second composition being such that the net rates of removal of water and di-alkyl carbonate from the vessel are approximately equal to their rate of formation whereby

(a) the water content of the vessel contents is maintained at not more than 5% by weight, and

(b) the volume of liquid in the vessel remains substantially constant,

said alcohol being introduced substantially at the rate at which it is consumed so that the average alcohol content of the vessel contents is maintained at not more than 25% by weight.

2. A process according to claim 1 wherein the first composition is removed from the vessel by condensation from the gas phase in the vessel.

3. A process according to claim 1 wherein the first composition is removed from the vessel as a part of the liquid phase in the vessel.

4. A process according to any one of claims 1 to 3 wherein an alcohol containing fraction is obtained by distillation of said first composition and said alcohol containing fraction is

recycled to the reaction vessel.

5. A process according to any one of claims 1 to 4 wherein said first composition is separated by distillation into a fraction richer in di-alkyl carbonate than said first composition and said di-alkyl carbonate richer fraction forms at least part of said second composition.

6. A process according to any one of claims 1 to 4 wherein said first composition is separated into a di-alkyl carbonate rich fraction and a water rich fraction by gravity separation and said second composition comprises part of said di-alkyl carbonate rich fraction.

7. A process according to any one of claims 1 to 6 wherein the alcohol content of said vessel contents is maintained at not more than 5% by weight.

8. A process according to any one of claims 1 to 7 wherein the water content of said vessel contents is maintained at not more than 1% by weight.

9. A process according to any one of claims 1 to 8 wherein the alcohol contains 1 to 5 carbon atoms.

10. A process according to claim 9 wherein the alcohol is methanol.

0134668

European Patent Office

# EUROPEAN SEARCH REPORT

Application number EP 84304796.0

## DOCUMENTS CONSIDERED TO BE RELEVANT

| Category | Citation of document with indication, where appropriate, of relevant passages | Relevant to claim | CLASSIFICATION OF THE APPLICATION (Int. Cl 4) |
| --- | --- | --- | --- |
| A | US - A - 4 360 477 (J.E. HALLGREN et al.)<br>* Example 17; claims *<br>---- | 1,9,10 | C 07 C 69/96<br>C 07 C 68/00 |
| | | | **TECHNICAL FIELDS SEARCHED (Int. Cl.4)** |
| | | | C 07 C 68/00<br>C 07 C 69/00 |

The present search report has been drawn up for all claims

| Place of search | Date of completion of the search | Examiner |
| --- | --- | --- |
| VIENNA | 06-11-1984 | HOFBAUER |

CATEGORY OF CITED DOCUMENTS

X : particularly relevant if taken alone
Y : particularly relevant if combined with another document of the same category
A : technological background
O : non-written disclosure
P : intermediate document
T : theory or principle underlying the invention
E : earlier patent document, but published on, or after the filing date
D : document cited in the application
L : document cited for other reasons
& : member of the same patent family, corresponding document

EPO Form 1503 03 82